# EUROPÄISCHE PATENTSCHRIFT

(11) **EP 1 361 418 B1**
(45) Veröffentlichungstag und Bekanntmachung des Hinweises auf die Patenterteilung: **20.07.2005**
(21) Anmeldenummer: 03405191.2
(22) Anmeldetag: 19.03.2003
(51) Int. Cl.: G01G 17/00, G01N 3/04, G01N 33/15

(54) **Vorrichtung zum Ausrichten von Tabletten**
Tablet alignment apparatus
Appareil à aligner les comprimés

(30) Priorität: 07.05.2002 CH 7702002
(43) Veröffentlichungstag der Anmeldung: 12.11.2003
(73) Patentinhaber: SOTAX AG, CH-4123 Allschwil (CH)
(72) Erfinder: Kalbermatten, Gilles, 68128 Roseau (FR)
(74) Vertreter: Eder, Carl E.

(56) Entgegenhaltungen:
- EP-A- 0 822 411
- DE-A- 4 241 985
- DE-C- 10 024 970
- US-B1- 6 260 419

## Beschreibung

Die Erfindung betrifft eine Vorrichtung zum Ausrichten von Tabletten, insbesondere Oblong-Tabletten und anderen nicht gleichförmigen Tabletten, an einer Mess-Station eines Tablettenprüfgerätes mit einem ebenen Transfertisch.

Tablettenprüfgeräte werden vorwiegend in der pharmazeutischen Industrie sowie auch in der Wissenschaft und Forschung zur Qualitätskontrolle und Produktcharakterisierung eingesetzt.

Bei der Entwicklung und besonders bei der routinemässigen Qualitätskontrolle von festen Arzneimittelformen sind die Bestimmungen von Gewicht, Dicke, Länge und Härte von grosser Bedeutung. Aus diesem Grund wurden bereits verschiedenste Prüfgeräte entwickelt, die mittels mehreren Mess-Stationen die Bestimmung von diesen physikalischen Parametern erlauben.

Bei bekannten Prüfgeräten gelangen die Tabletten von einer Zufuhreinrichtung auf eine Transporteinrichtung mit einem Rechen der mehrere Gabeln aufweist, die die Tabletten auf einer Transportbahn weiterbewegen. Eine Bewegungseinrichtung für den Rechen transportiert dabei den Rechen um eine Strecke in Richtung der Transportrichtung, hebt dann den Rechen an und führt ihn in angehobenem Zustand entgegen der Transportrichtung um eine entsprechende Strecke wieder zurück. Anschliessend wird der Rechen abgesenkt und erneut um eine Transportstrecke weiterbewegt. Ferner sind entlang der Bewegungsbahn des Rechens mehrere Teststationen, nämlich eine Waage, eine Teststation für die Dickenmessung und eine Teststation für den Bruchtest vorgesehen. Die meisten Tablettenprufgeräte sind für runde oder kugelförmige Tabletten geeignet, weniger jedoch fur Oblong-Tabletten und ähnlich geformte Prüflinge, die eine im wesentlichen ovale Form in Draufsicht, einen flachen Steg an ihrem Umfang sowie eine gewölbte Ober- und Unterseite aufweisen. Derartige Prüflinge bereiten auf diesen Geräten vermehrt Schwierigkeiten. Sie können nämlich in den verschiedenen Teststationen und vor allem in der Station zur Bruchhärteprüfung nicht immer in der erforderlichen Art und Weise optimal ausgerichtet werden.

Ein Tablettenprüfgerät, das zum Positionieren von Oblong-Tabletten oder ahnlich geformten Prüflingen in Messstationen eines Prüfgerätes geeignet ist, ist in der deutschen Offenlegungsschrift 197 33 436 beschrieben. Das darin offenbarte Tablettenprüfgerät zeichnet sich durch eine Zufuhreinrichtung zur Zufuhr einzelner Prüflinge an eine Waage aus, deren Waagschale einen in Transportrichtung geneigten Boden in Form einer Rinne aufweist. Eine Transporteinrichtung unterhalb der Waagschale, auf die die Prüflinge von der Waagschale mit ihrer Längsachse parallel zur Längsrichtung der Rinne abgelegt werden, transportiert die Prüflinge in dieser Ausrichtung zu den Teststationen.

Auch dieses bekannte Gerät löst das Problem der horizontalen Ausrichtung und Positionierung der Prüftabletten nicht ohne Nachteile. So erlaubt dieses Gerät keine optimale Ausrichtung des Prüflings direkt an der zum Beispiel dem Härtetest dienenden Messstation, was immer wieder zu Fehlmessungen und Störungen des Prüfgerätes führt.

Die Härte eines Prüflings wird üblicherweise in einer fein auflösenden Krafmessdose gemessen, die einen feststehenden Anschlag und einen beweglichen Pressbacken aufweist. Der Prüfling wird in den Bereich zwischen Anschlag und Pressbacken auf eine Führungsbahn befordert, wobei der Prüfling vorzugsweise den Anschlag berührt. Der Pressbacken wird nun mittels eines Schrittmotors gegen den Anschlag und den vor diesem liegenden Prüfling gefahren. Die vom Pressbacken mit jedem Schritt des Motors ausgeübte Kraft wird gemessen und aufgezeichnet, die konstant und sehr klein ist, solange der Anschlag den Prüfling nicht berührt oder dieser ohne Gegendruck über die Führungsbahn geschoben wird. Wenn der Pressbacken den Prüfling gegen den Anschlag drückt, steigt die von ihm ausgeübte Kraft mit jedem Schritt des Schrittmotors so lange an, bis der Prüfling zerbricht. Die dazu aufgewendete Kraft wird aufgezeichnet und dient als Mass für die Härte des Prüflings. Das plötzliche Abfallen der vom Pressbacken aufgewendeten Kraft beim Zerbrechen des Prüflings dient ferner als Abbruchbedingung für das Beenden der Messung. Der Pressbacken wird in seine Ausgangsposition zurückgefahren und der nächste Prüfling kann geprüft werden.

In einem speziell dem Härtetest dienenden und aus der deutschen Offenlegungsschrift 100 24 970 bekannten Prüfgerät wird unter Mitnahme des Prüflings die Vorschubbewegung des beweglichen Pressbackens zum Transport des Prüflings in Richtung des feststehenden Anschlags fortlaufend unterbrochen und der Pressbacken zurückgezogen und vorgeschoben, wobei die Vorschub- und Ruckschubbewegung des Pressbackens in Bezug auf seinen gesamten Vorschubweg bezüglich des Anschlags klein ist, so dass der vorzugsweise in einer Rinne geführte Prüfling in seine Ruhelage zurückschwingt und danach der Pressbacken wieder zum Schub in Richtung des Anschlags ansetzt, bis der Prüfling den Anschlag erreicht hat. Dieses Verfahren erlaubt zwar die Durchfuhrung eines Härtetests an einem Prüfling der wenigstens eine gewölbte Hauptoberfläche aufweist, nicht aber eine störungsfreie Messung von Oblong-Tabletten.

In der US-A-6,260,419 ist ein Härteprufgerät für Tabletten oder Pillen beschrieben. Um zu erreichen, dass Kraftwirkung auf das Testobjekt immer in einer definierten optimalen Achse des Testobjekts erfolgt, werden bei dieser Vorrichtung nicht die Testobjekte ausgerichtet, sondern die sich zufällig ergebende Ausrichtung jedes einzelnen Testobjekts bestimmt und danach die Richtung der Pressbacken entsprechend der Ausrichtung des Testobjekts nachgeführt wird. Bei dieser Methode muss praktisch bei jedem Prüfobjekt eine Ausrichtung der Apparatur erfolgen. Dies ist ausserordentlich aufwendig.

Der vorliegenden Erfindung liegt nun die Aufgabe zu Grunde, eine neuartige Vorrichtung zur Verfugung zu stellen, die ein manuelles, halbautomatisches oder automatisches Ausrichten von in eine Mess-Station eines Tablettenprüfgerates eingebrachten Tabletten erlaubt, bei welcher die Messung mit ausreichender Genauigkeit kontrolliert werden kann, in verhaltnismässig kurzen Zeitabständen mit neuen Testtabletten wiederholt werden kann und gleichzeitig die Nachteile von in bekannten Prüfgeräten eingesetzten Ausrichtungsmitteln nicht aufweist.

Diese Aufgabe wird gelöst durch eine Vorrichtung, wie sie im Anspruch 1 definiert ist. Ebenfalls Gegenstand der vorliegenden Erfindung ist ein Tablettenprüfgerät, das mit einer Vorrichtung gemäss Anspruch 1 vorgesehen ist und die im Anspruch 4 aufgelisteten Merkmale besitzt.

Vorteilhafte Weiterbildungen der Vorrichtung und des Tablettenprüfgerätes gehen aus den abhangigen Ansprüchen hervor.

Die erfindungsgemässe Vorrichtung und das erfindungsgemässe Prüfgerät weisen verschiedene Vorzüge im Vergleich zum Stand der Technik auf. So ergibt die Erfindung den Vorteil, dass ohne Einsatz von horizontalen Ausrichtungselementen vor allem Oblong-Tabletten absolut genau und automatisch entlang ihrer eigenen Korperlängsachse ausgerichtet werden, sobald sie auf das Rollenpaar der erfindungsgemässen Vorrichtung zu liegen kommen und dass die der Ausrichtung dienenden horizontalen Rollen aufgrund von fehlenden verschiebbaren mechanischen Elementen wenig storungsanfällig sind. Deshalb sind die erfindungsgemässe Vorrichtung und das erfindungsgemässe Prüfgerät insbesondere auch gut geeignet zur Verwendung bei einer automatischen, halbautomatischen oder manuellen Härteprüfung von Tabletten mit einer Teilchenlänge von beispielsweise 5 mm bis 20 mm und einer mittleren Teilchenbreite von üblicherweise 4 mm bis 8 mm.

Im Folgenden werden Ausführungsbeispiele der Erfindung anhand einer Zeichnung beschrieben. In der Zeichnung zeigt
die Figur 1 eine perspektivische Teilansicht eines Tablettenprüfgerätes mit einer Vorrichtung zum Ausrichten von Testtabletten,
die Figur 2 eine Draufsicht auf die Vorrichtung gemäss Figur 1,
die Figur 3, einen Längsschnitt durch einen Teil des Tablettenprüfgerätes, und
die Figur 4 einen Schnitt entlang der Linien IV- IV der Figur 3.

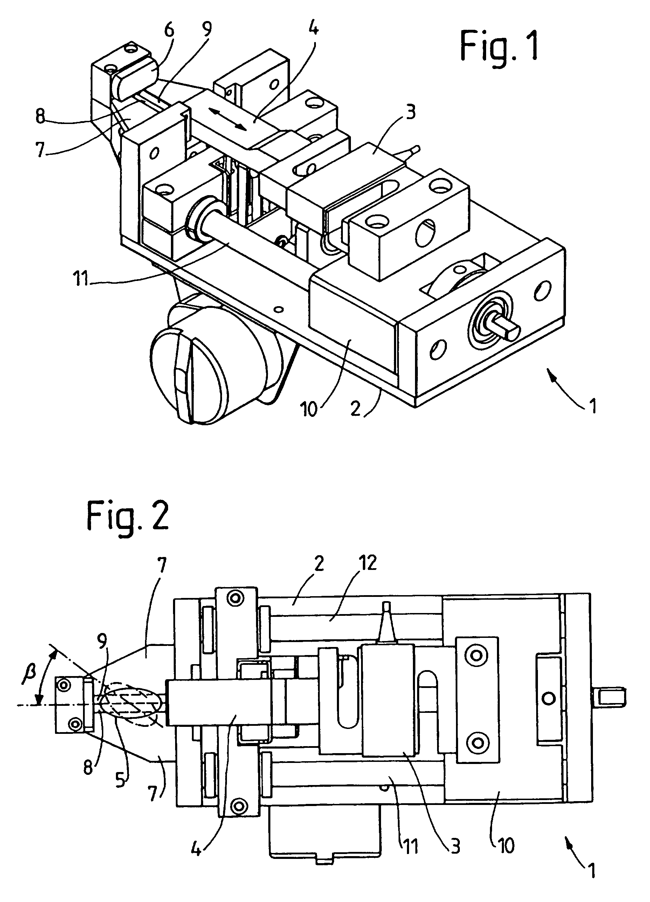

Das in den Figuren 1 bis 3 zum Teil dargestellte und als ganzes mit 1 bezeichnete Kopfteil eines Tablettenprüfgerätes besitzt eine auf einer horizontalen Grundplatte 2 angeordnete Kraftmessdose 3. Diese ist bekannter Bauart und besitzt einen Stossel 4 zum Brechen der zu prüfenden Tablette 5, wozu dem Stossel 4 noch zusätzlich ein Tabletten-Anschlag 6 zugeordnet ist.

In der zwischen Anschlag 6 und Stössel 4 vorgesehenen Ebene, welche durch einen der Tablettenführung dienenden, vorzugsweise horizontalen Transfertisch 7 gebildet wird, sind erfindungsgemäss zwei parallel nebeneinander angeordnete Rollen 8 und 9 zum Ausrichten der Prüftablette 5 vorgesehen. Die Rollen 8 und 9 sind insbesondere parallel zur Stossrichtung des Stössels 4 ausgerichtet, und es verläuft die Längsachse des Stössels 4 in einer sich genau zwischen den beiden Rollen 8 und 9 befindlichen Vertikalebene.

Die oberen Mantellinien der beiden vorzugsweise metallischen Rollen 8 und 9 sind mit der Auflagefläche des Transfertisches 7 bündig oder stehen leicht über diese hervor.

Die Rollen 8 und 9 besitzen beispielsweise einen Durchmesser von je 3 mm bis 5 mm, vorzugsweise 4 mm, und einen Achsenabstand von 3,5 mm bis 6 mm, vorzugsweise 4,4, mm. Sie sind zudem mit einer Antriebsvorrichtung 10 verbunden und werden über Wellen 11 und 12 in Pfeilrichtung 13 bzw. 14 gegenläufig angetrieben, und zwar so, dass die linke Rolle im Uhrzeigersinn und die rechte Rolle gegen den Uhrzeigersinn gedreht werden.

Gelangt nun eine Prüftablette 5, beispielsweise eine Oblong-Tablette, vom Transfertisch 7 auf die beiden sich drehenden Rollen 8 und 9, so richtet sich diese um den Winkel β automatisch nach den Achsen der beiden Rollen 8 und 9 aus, dass heisst es wird die Körperlangsachse nach den beiden Rollenachsen ausgerichtet, und dies unabhängig davon, wie ihre Lage zu den Rollenachsen ursprünglich war. Die Tablette 5 lässt sich also erfindungsgemäss relativ einfach und ohne horizontal bewegliche Elemente in einer bestimmten Achse ausrichten, und so in optimaler Lage dem darauffolgenden, eingangs bereits beschriebenen, Härtetest unterziehen, auf welchen nachfolgend nicht näher eingegangen wird.

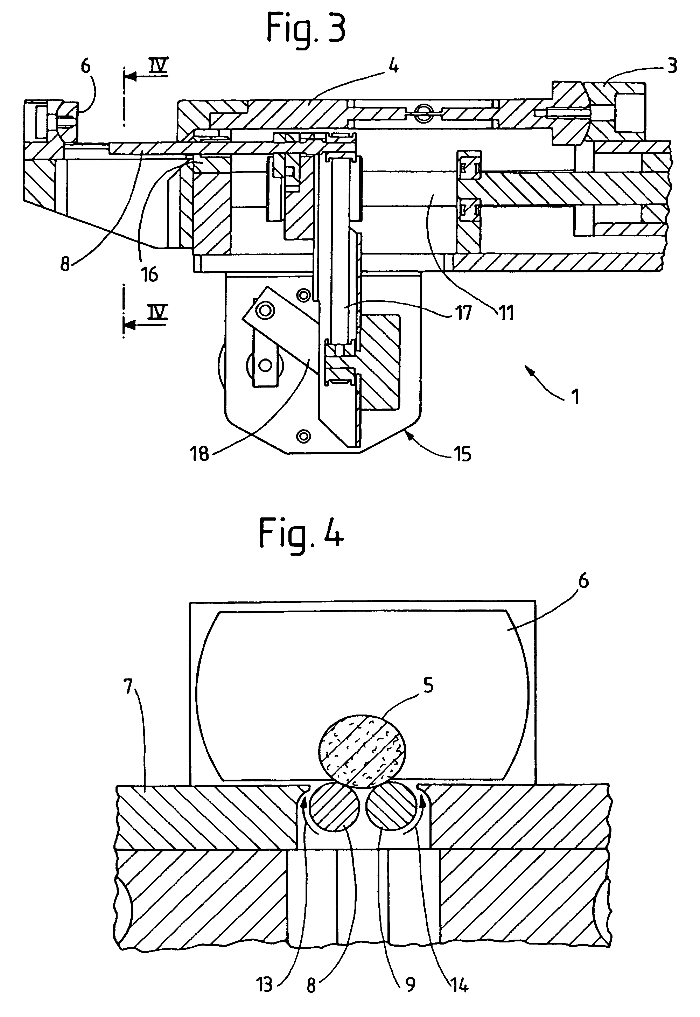

Im in der Figur 3 dargestellten Schnitt sind die Rollenpaare 8 und 9 gegenüber dem Betriebszustand leicht zurückversetzt dargestellt. Diese sind nämlich noch zusätzlich mittels einer weiteren Antriebsvorrichtung 15 horizontal verschiebbar, und zwar so, dass sie beispielsweise zu Reinigungszwecken durch Abstreifmittel 16 hindurchführbar sind. Die Bewegung kann dabei über Antriebsmittel 17 und 18 so erfolgen, dass diese ungleichförmig ist, dass heisst beim Zurückfahren langsam beginnt, dann schneller wird, in der vollständig zurückgefahrenen Position die hochste Geschwindigkeit besitzt und beim Vorwärtsverschieben wieder kontinuierlich an Geschwindigkeit abnimmt. Insbesondere beim Zurückfahren der Rollenpaare 8 und 9 fallen die Tablettenbruchstücke vom Transfertisch 7 vertikal hinunter in einen nicht gezeichneten Abfallbehälter, so dass dadurch die Ebene des Transfertisches 7 automatisch gesäubert wird.

Die erfindungsgemässe Vorrichtung ist übrigens keineswegs beschränkt auf Anwendungen fur den Härtetest von pharmazeutischen Tabletten. Es können ohne weiteres auch Prüfgeräte zum Test von anderen festen Produkten, wie Wasch- oder Düngemitteltabletten, mit der erfindungsgemässen Vorrichtung zur Ausrichtung von Prüflingen ausgestattet sein und/oder es kann das Prüfgerät anstelle einer Kraftmessdose zur Härtebestimmung oder noch zusätzlich dazu mit Messmitteln zur Bestimmung von Länge, Gewicht und mittlerer Breite eines Prüflings versehen sein.

## Patentansprüche

1. Vorrichtung zum Ausrichten von Tabletten (5) an einer Mess-Station eines Tablettenprüfgerätes, mit einem ebenen Transfertisch (7) **gekennzeichnet durch**
- zwei in der Auflageebene des Transfertisches (7) vorgesehene, parallel nebeneinander angeordnete Rollen (8, 9), wobei die oberen Mantellinien der Rollen (8, 9) mit der genannten Auflageebene bundig sind oder leicht über diese vorstehen, und
- eine Antriebsvorrichtung (10), welche beim Betrieb der Vorrichtung die beiden Rollen (8, 9) so gegenläufig antreibt, dass die linke Rolle im Uhrzeigersinn und die rechte Rolle gegen den Uhrzeigersinn gedreht werden, so dass sich eine auf die beiden Rollen (8, 9) transferierte Testtablette (5) automatisch nach den Achsen der beiden Rollen (8, 9) ausrichtet.

2. Vorrichtung nach Anspruch 1, **dadurch gekennzeichnet, dass** die beiden Rollen (8, 9) aus metallischem Material bestehen und einen Durchmesser von je 3 mm bis 5 mm, vorzugsweise 4 mm, und einen Achsenabstand von 3,5 mm bis 6 mm, vorzugsweise 4,4 mm, besitzen.

3. Vorrichtung nach Anspruch 1 oder 2, **dadurch gekennzeichnet, dass** die beiden Rollen (8, 9) horizontal verschiebbar sind und dass Abstreifmittel (16) vorhanden sind, durch welche die Rollen (8, 9) beim horizontalen Verschieben hindurchgeführt werden.

4. Tablettenprüfgerät mit wenigstens einer Mess-Station zur Bestimmung mindestens eines physikalischen Parameters einer Prüftablette (5), **gekennzeichnet durch** eine Vorrichtung gemäss einem der Ansprüche 1 bis 3.

5. Tablettenprüfgerät nach Anspruch 4, **dadurch gekennzeichnet, dass** sie eine der Hartebestimmung dienende Kraftmessdose (3) besitzt, die einen Stössel (4) zum Brechen der zu prüfenden Tablette (5) und einen dem Stössel (4) gegenüberliegenden Tabletten-Anschlag (6) aufweist, dass in einer zwischen Anschlag (6) und Stössel (4) vorgesehenen Ebene die zwei parallel nebeneinander angeordnete Rollen (8, 9) vorgesehen sind, wobei die Längsachse des Stössels in einer sich genau zwischen den beiden Rollen (8, 9) befindlichen Vertikalebene liegt.

## Claims

1. Apparatus for aligning tablets (5) on a measuring station of a tablet tester having a flat transfer table (7), **characterized by**
- two rollers (8, 9) provided in the support plane of the transfer table (7) and arranged parallel to one another, the upper generating lines of the rollers (8, 9) being flush with said support plane or projecting slightly above it, and
- a drive device (10) which, during operation of the apparatus, drives the two rollers (8, 9) in opposite directions so that the left roller is rotated clockwise and the right roller is rotated counterclockwise, so that a test tablet (5) transferred onto the two rollers (8, 9) automatically aligns with the axes of the two rollers (8, 9).

2. Apparatus according to Claim 1, **characterized in that** the two rollers (8, 9) consist of metallic material and each have a diameter of 3 mm to 5 mm, preferably 4 mm, and an axial spacing of 3.5 mm to 6 mm, preferably 4.4 mm.

3. Apparatus according to Claim 1 or 2, **characterized in that** the two rollers (8, 9) are horizontally displaceable, and **in that** scraping means (16) are present, through which the rollers (8, 9) are moved on horizontal displacement.

4. Tablet tester comprising at least one measuring station for determining at least one physical parameter of a test tablet (5), **characterized by** an apparatus according to any of Claims 1 to 3.

5. Tablet tester according to Claim 4, **characterized in that** it has a load cell (3) which serves for hardness determination and comprises a ram (4) for breaking the tablet (5) to be tested and a tablet stop (6) opposite the ram (4), and **in that** the two rollers (8, 9) arranged parallel to one another are provided in a plane provided between stop (6) and ram (4), the longitudinal axis of the ram being in a vertical plane present exactly between the two rollers (8, 9).

## Revendications

1. Dispositif d'alignement de comprimés (5) dans un poste de mesure d'un appareil de contrôle de comprimés équipé d'un plateau de transfert (7) plan, **caractérisé par**
- deux rouleaux (8, 9) agencés en juxtaposition parallèle et prévus dans le plan d'appui du plateau de transfert (7), les génératrices supérieures desdits rouleaux (8, 9) étant situées dans l'affleurement du plan d'appui précité, ou faisant légèrement saillie au-dessus de ce dernier, et
- un dispositif d'entraînement (10) qui, lors du fonctionnement du dispositif, entraîne les deux rouleaux (8, 9) dans des sens opposés, de façon que le rouleau de gauche soit animé d'une rotation dans le sens horaire, et qu'une rotation dans le sens antihoraire soit imprimée au rouleau de droite, de telle sorte qu'un comprimé d'expérimentation (5), transféré sur les deux rouleaux (8, 9), s'aligne automatiquement avec les axes des deux rouleaux (8, 9).

2. Dispositif selon la revendication 1, **caractérisé par le fait que** les deux rouleaux (8, 9) sont constitués d'un matériau métallique et possèdent un diamètre mesurant respectivement de 3 mm à 5 mm, de préférence 4 mm, ainsi qu'un entraxe de 3,5 mm à 6 mm, de préférence 4,4 mm

3. Dispositif selon la revendication 1 ou 2, **caractérisé par le fait que** les deux rouleaux (8, 9) peuvent coulisser horizontalement ; et par la présence de moyens de raclement (16) à travers lesquels lesdits rouleaux (8, 9) sont guidés lors du coulissement horizontal.

4. Appareil de contrôle de comprimés, équipé d'au moins un poste de mesure pour déterminer au moins un paramètre physique d'un comprimé d'expérimentation (5), **caractérisé par** un dispositif conforme à l'une des revendications 1 à 3.

5. Appareil de contrôle de comprimés, selon la revendication 4, **caractérisé par le fait qu'**il possède une boîte dynamométrique (3) servant à déterminer la dureté, et comprenant un coulisseau (4) pour rompre le comprimé (5) à contrôler, et un arrêtoir (6) de comprimés qui est situé en vis-à-vis dudit coulisseau (4) ; et **par le fait que** les deux rouleaux (8, 9) agencés en juxtaposition parallèle sont prévus dans un plan prévu entre l'arrêtoir (6) et le coulisseau (4), l'axe longitudinal dudit coulisseau se trouvant dans un plan vertical situé exactement entre les deux rouleaux (8, 9).
